# EUROPEAN PATENT SPECIFICATION

(11) **EP 1 755 466 B1**
(45) Date of publication and mention of the grant of the patent: **19.12.2007**
(21) Application number: 05749593.9
(22) Date of filing: 14.06.2005
(51) Int. Cl.: A61B 17/15

(54) **AN IMAGELESS ROBOTIZED DEVICE FOR SURGICAL TOOL GUIDANCE**
BILDLOSE ROBOTERVORRICHTUNG ZUR FÜHRUNG EINES CHIRURGISCHEN WERKZEUGS
DISPOSITIF ROBOTISE SANS IMAGE POUR LE GUIDAGE D'UN INSTRUMENT CHIRURGICAL

(30) Priority: 15.06.2004 FR 0406491
(43) Date of publication of application: 28.02.2007
(73) Proprietor: Zimmer GmbH, 8404 Winterthur (CH)
(72) Inventor: NAHUM, Bertin Résidence "Le Séville", F-34000 Montpellier (FR); TASSEL, Eric, F-34000 Montpellier (FR); BLONDEL, Lucien, F-34000 Montpellier (FR); MAILLET, Pierre, F-11000 Carcassonne (FR)
(74) Representative: Manitz, Finsterwald & Partner GbR
(86) International application number: PCT/EP2005/052751
(87) International publication number: WO 2005/122916

(56) References cited:
- US-A- 4 549 540
- US-A- 4 913 413
- US-A- 5 403 319
- US-A- 5 871 018
- US-A1- 2003 130 665
- US-A1- 2004 111 183
- KIENZLE T C ET AL: "TOTAL KNEE REPLACEMENT" IEEE ENGINEERING IN MEDICINE AND BIOLOGY MAGAZINE, IEEE INC. NEW YORK, US, vol. 14, no. 3, 1 May 1995 (1995-05-01), pages 301-306, XP000505086 ISSN: 0739-5175

## Description

### FIELD OF THE INVENTION

The present invention relates to the field of robotic-aided surgical systems. It applies in particular to mechanical guidance for an oscillating saw blade or a drill in a variety of surgical applications. For instance, in a total knee replacement surgery, the present invention improves the accuracy of implant installation and its longevity providing a reliable guidance system.

### BACKGROUND OF THE INVENTION

Many surgical procedures in various specialities (orthopaedics, neurosurgery, maxillofacial, etc.) require precise bone cutting or drilling. It is the case for example for surgeries around the knee (knee arthroplasty, tibial or femoral osteotomy, ligamentoplasty), in spine surgery (pedicular screws placement) or in neurosurgery.

These procedures are traditionally carried out using motorized instruments (surgical drill, oscillating saw, etc.) positioned and maintained either directly by the surgeon or using basic mechanical guides.

However, there are many studies in the literature showing that existing techniques do not guarantee a good and foreseeable result. They suggest that a more precise execution of cuts and drillings would lead to better post-operative results.

It would be desirable to provide improved systems and methods for performing surgical gestures that would perfectly match surgeon's operative plans. Crucial issues in such surgical gestures include the necessity to obtain perfect alignments of cuts or drillings with respect to patient anatomy as well as relative alignments of cuts or drillings.

Total knee replacement (TKR) is an example of a surgical procedure that requires accurate cuts. In TKR, the surgeon resects the distal femur and the proximal tibia and replaces them with prosthetic components to restore correct functionality of the knee. Theses components have to be properly aligned with respect to the mechanical axes of the bones. Otherwise, the result can lead to poor knee kinematics or loosening of the components. Misalignment can occur in many different ways: orientations along three axes (varus/valgus, flexion/extension, internal/external) and translation along three axes (medial/lateral, proximal/distal, anterior/posterior). Currently, conventional TKR involves a complex jig system of cutting blocks and alignment rods. It is difficult for the surgeon to correctly position the cutting blocks with alignment rods laid along the estimated axes.

There are evidence in the literature that theses techniques are not satisfactory. According to studies such as "Navigation in total-knee arthroplasty: CT-based implantation compared with the conventional technique.", Perlick L, et al., Acta Orthop Scand. 2004, vol. 4, pp 464-470, and "The effect of surgeon experience on component positioning in 673 PFC posterior cruciate-sacrificing total knee arthroplasties", by Mahaluxmivala J, et al., J. Arthroplasty 2001, vol.5, pp 635-640, almost one third of such operations are outside the alignment limits (between 3 degrees varus and 3 degrees valgus from ideal postoperative leg axis). Perlick L., et al., in "Useability of an image based navigation system in reconstruction of leg alignment in total knee arthroplasty", Biomed Tech (Berlin) 2003, vol. 12, pp 339-343, found in a study of 50 knees that only 70 percent were inside the alignment limits. Conventional instrumentation is of some assistance to the surgeon in achieving the correct alignment between the leg axis and the implant but the result depends highly on the surgeon's experience.

Different approaches have been proposed to assist the surgeon during TKR. Navigation systems are based on a tracking system that locates the spatial position of trackers. Trackers are fixed on the femur, on the tibia and on mechanical devices such as cutting blocks and pointing tools. The surgeon can visually follow the relative position of the tool with respect to the bones. In a first step, the surgeon registers anatomical landmarks and surfaces with a tracked pointer and defines the center of the hip joint by a kinematic procedure. The navigation system is then able to compute the mechanical axes of the bones and the optimal position for the different cuts. Implanting pins, the surgeon fixes the cutting blocks on the bone with the visual help provided by the navigation system. Drawbacks of such systems are their complexity, the longer procedure time required, and their lack of assistance for the actual surgical gesture realization. There can also be significant loss of accuracy in the positioning of cutting blocks at the very moment when the surgeon looks away from the navigation system screen to implant the fixation pins. Therefore, these navigated solutions still mainly rely on surgeon's skill.

Robotic systems have also been proposed to improve bone cutting during knee replacement surgery. T.C. Kienzle in 'Total Knee Replacement', IEEE Engineering in Medicine and Biology, vol. 14, no. 3, 1995-05-01, describes a computer-assisted surgical system using a calibrated robot. The system uses a workstation which displays a 3D model of the patient's bones obtained from a CT scan of the leg and a modified industrial robot which directs the placement of prosthetic components. Positions of fiducial markers fixed on the bones are measured with a probe attached to the robot mounting flange. They serve to register the preoperative image data (CT scan frame) with the position of the patient (robot reference frame). After computing the optimal placement of the prosthesis component, the robot positions a drill guide where the holes for the cutting block are to be placed. Main drawback of this system is that surgeon has to perform a pre-operative surgical procedure to place invasive pins in the patient's femur and tibia before carrying out a CT-scan of the leg. In this system, no pointing tool received by the robot arm to acquire the coordinates of anatomical landmarks and no means for manually acquiring and for memorizing the coordinates of the anatomical landmarks are provided.

Another robotic device is disclosed in U.S Pat. No. 5,403,319. This device comprises a bone immobilization device, an industrial robot and a template attached to the robot mounting flange. The template has a functional interior surface corresponding to the exterior surface of the femoral component of a knee prosthesis. In the first step, the surgeon positions the template in the desired position of the prosthesis and the robot registers the position. In the second step, the system combines the registered position with a geometric database to generate coordinate data for each cutting task. The robot then positions a tool guide perfectly aligned for each specific task. The actual surgical task is carried out by the surgeon through the tool guide. One of the main drawbacks of this system is that its accuracy entirely relies on an unlikely hypothesis: surgeon's ability to determine visually the optimal spatial position of the prosthesis. Practically, it is almost impossible even for a high-skilled surgeon to position freehand a prosthesis template with an accuracy sufficient to obtain a good post-operative result. Authors describe some rudimentary alignment means such as cut guide marks, alignment tabs and reference rods that could be used for evaluating the position and orientation of the prosthesis relative to the bone. These means are far less accurate than conventional instrumentation. Therefore, this system would be certainly less accurate than conventional jig systems. Another main drawback is that this system anticipates one prosthesis template for each type and size of implant component. As there are around a hundred different models of prosthesis commercialized and around 5 to 7 sizes for each model, this solution seems rather unadapted to operating room constraints.

Other robotic systems have been proposed for performing total knee replacement, many of them using pre-operative image data of the patient. ROBODOC (TM) and CASPAR (TM) surgical systems are active robots that mill automatically the bones, realizing autonomously the surgical gesture. The Acrobot (TM) surgical system is a semi-active robot assisting the surgeon during the milling. All these systems are image based.

Other automated systems are proposed in combination with a navigation system. It is the case for the Praxiteles (TM) device from PRAXIM, the Galileo (TM) system from Precision Implants and the GP system (TM) from Medacta International (TM). All these systems are bone-mounted, requiring a large incision, and cannot work without a navigation system.

Other surgeries around the knee like tibial osteotomy and ligament repairs share the same issues as TKR: accurate cuts or drillings are required to restore knee functionality. In a tibial osteotomy for example, a bone wedge is removed from the tibia to change the axis of the bone. The angular correction is determined pre-operatively on an X-ray. As for TKR, conventional instrumentation includes very basic mechanical guides. There is a need for assistance in precise bone cut.

### SUMMARY OF THE INVENTION

The present invention provides an imageless system for surgical tool guidance by accurately positioning a guide mounted to a robot arm, typically a cutting guide used in knee replacement surgery for guiding an oscillating saw.

The method of using it comprises the steps of: collecting anatomical landmarks with a robot arm; combining landmarks data with geometric planning parameters to generate a position data; automatically positioning a tool guide mounted to the robot arm.

In one preferred embodiment, the device is a robotized surgical device used for the optimal positioning of a cutting or drilling guide.

The robotized device is rigidly attached to the operating table by a specific fixation device.

Preferably, the robot arm presents at least six degrees of freedom and is adapted to receive a cutting and/or drilling guide and/or a pointing tool. Same instrument can be used both for pointing and guiding.

The robotized device accurately positions the guide at the place where cutting or drilling must be carried out. Bone cutting or drilling is realized through the guide by a surgeon using an oscillating saw or a surgical drill,

In one preferred embodiment, the robot arm comprises a force sensor and can work in a cooperative mode in which the user has the ability to move the robot arm manually by grabbing it by its final part.

In another preferred embodiment, movements of the guide in the cooperative mode can be restricted either to a plane for a cutting guide or to an axis for a drilling guide.

In another preferred embodiment, the system such as briefly exposed above comprises a display monitor provided with a user communication interface to receive planning parameters from a user.

Anatomical landmarks data and planning parameters are combined to define the optimal position of the guide. For example, in TKR, the internal rotation of the femoral component is a planning parameter for implant positioning. The user communication interface could be, for example, a keyboard, a touch screen and/or a mouse.

In another embodiment, the device also comprises an interface with a surgical navigation system being able to work **from** intra-operative data. Data provided by the surgical navigation system are then used to generate position data for the guide. In this case, the use of a navigation system supplements the step of collecting anatomical landmarks with the robot. Data is provided from the navigation system through a communication interface in accordance to a predefined protocol. The robotized device object of the invention is then a peripheral for precise execution of the surgical planning realized by means of the surgical navigation system.

Preferably, the guiding tool comprises limited surfaces to reduce contact and friction with an oscillating saw while preserving an efficient guidance.

In another preferred embodiment, the robotized device comprises a limb fixation device adapted to ensure immobilization of the leg at two levels: at the level of the ankle with a toothed rack; at the level of the knee with two pins screwed in the femoral or tibial epiphysis.

These means of fixation of the limb ensure the immobility of the leg during the steps of anatomical landmarks collection and bone cutting and/or drilling.

Other advantages, goals and characteristic of this invention will arise from the following description.

### BRIEF DESCRIPTION OF THE DRAWINGS

For a better understanding of the nature, objects and function of the present invention, reference should be made to the following detailed description in conjunction with the accompanying drawings, in which:
FIG. **1** is an overview of the system of the present invention showing a mobile base, a robot arm with a force sensor and a tool mounted on, and a display monitor;
FIG. **2A** is a perspective view of the pointing tool;
FIG. **2B** is a perspective view of the guiding tool;
FIG. **2C** is a perspective view of a pointing and guiding tool;
FIG. **3** is a perspective view of a fixation device for rigidly fixing the mobile base to the operating table;
**FIG. 4A** is a perspective view of a limb fixation device that rigidly holds the leg to the operating table;
FIG. **4B** is a perspective view of the plate of the limb fixation device described in FIG. 4A;
FIG. **4C** is a perspective view of the knee part of the limb fixation device described in FIG. 4A;
FIG. **4D** is a perspective view of the ankle part of the limb fixation device described in FIG. 4A;
FIG. **5** is an exploded view of the pointing tool, the force sensor and the robot arm mounting flange;
FIG. **6** is an overview of the system of the present invention including a patient positioned on an operating table; and
FIG. **7** is a block diagram showing various modules of the control software.

### DETAILED DESCRIPTION OF THE SPECIFIC EMBODIMENTS

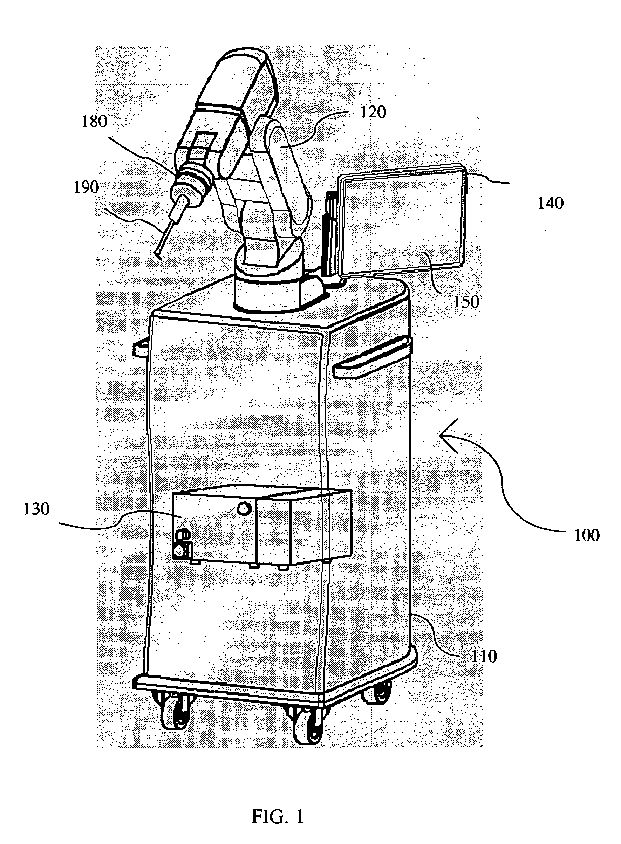

With reference to **FIG. 1,** it can be seen that a preferred embodiment of the present invention generally includes a robotized device **100** comprising a mobile base **110;** a robot arm **120;** a control unit **130** inside the mobile base, that controls the robot arm **120** and allows a surgeon to manually input data through the use of an interface **150** that can be a touch screen, a mouse, a joystick, a keyboard or the like; a display monitor **140;** a tool **190** and a force sensor **180** mounted to the robot arm mounting flange; and specific fixation device **170** to fix the robotized device **100** to an operating table (not represented here).

Mobile base **110** ensures easy handling of the robotized device **100** with its wheels and handles. Mobile base **110** is also preferably provided with immobilization pads or equivalent.

Robot arm **120** is a six joint arm. Each joint is provided with an encoder which measures its angular value. These data, combined with the known geometry of the six joints, allow to compute the position of the robot arm mounting flange and the position of the tool mounted to the robot arm, either a pointing tool, a guiding tool or a pointing and guiding tool.

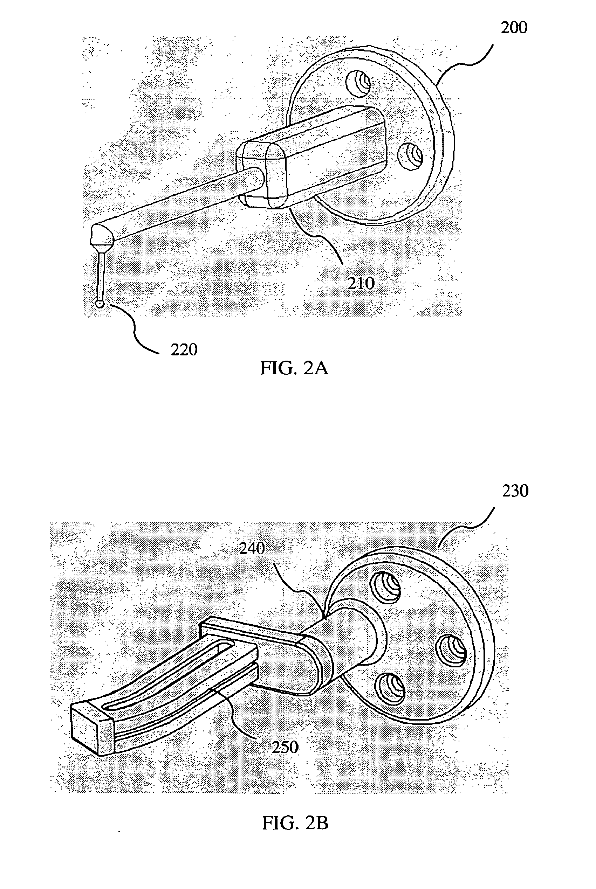

**FIG. 2A** illustrates a pointing tool **190.** The pointing tool **190** comprises a base plate **200;** a handle **210;** and a pointing sphere **220.**

**FIG. 2B** illustrates a cutting guide. The cutting guide comprises a base plate **230;** a handle **240** and a slit **250** to guide a saw blade.

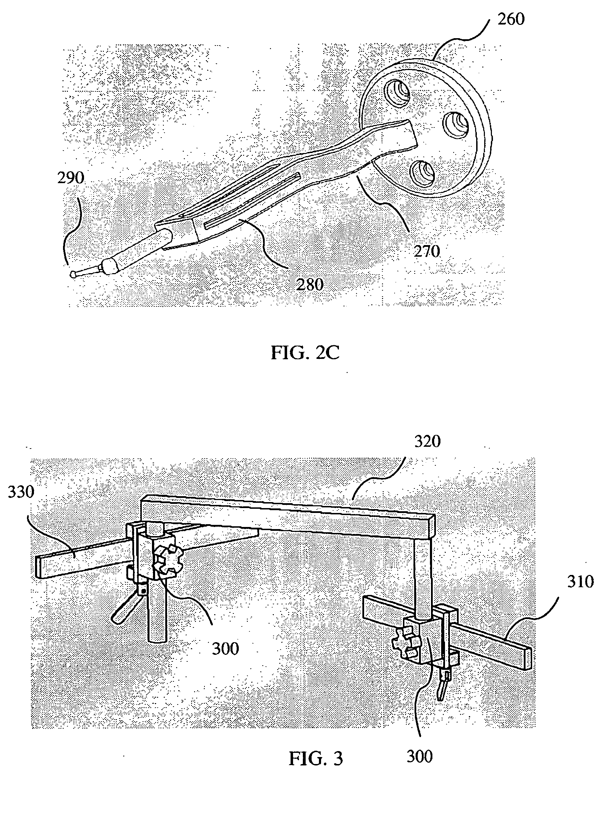

**FIG. 2C** illustrates a pointing and guiding tool. It comprises a base plate **260;** a handle **270;** a slit **280** to guide a saw blade and a pointing sphere **290.**

The tools described in **FIGS. 2A** to **2C** are just three examples of pointing and/or guiding tools that may be utilized with the device shown in **FIG. 1.**

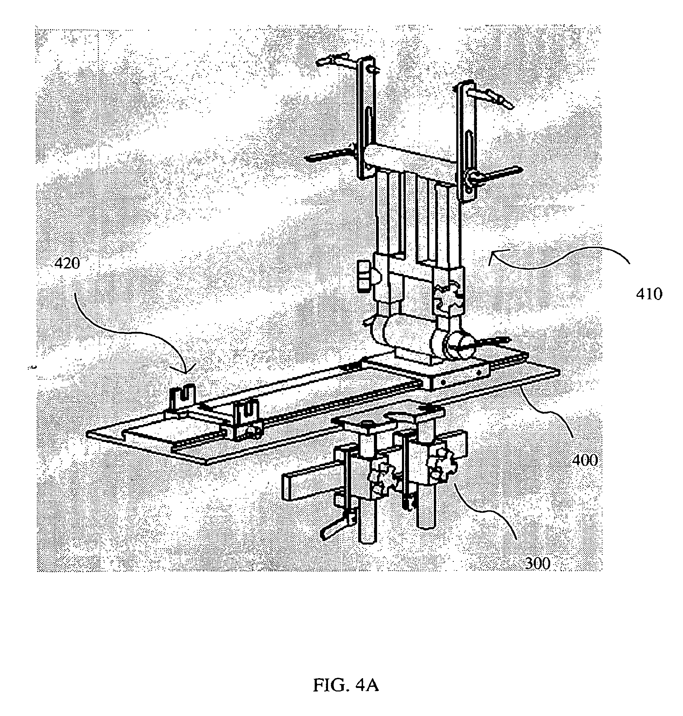

Preferably, robot arm **120** is rigidly attached to the operating table by a specific base fixation device. As shown in **FIG. 3,** a base fixation device includes two sets of clamps **300** adapted to the operating table rail **310** and U-shape bars **320.** Initially, the user installs one clamp **300** on the operating table rail **310** and another clamp on the mobile base rail **330.** When clamps are in place, the user inserts the U-shape bar in the cylindrical holes of the clamps, locks the clamps in place and locks the U-shape bar inside the clamps using the knobs.

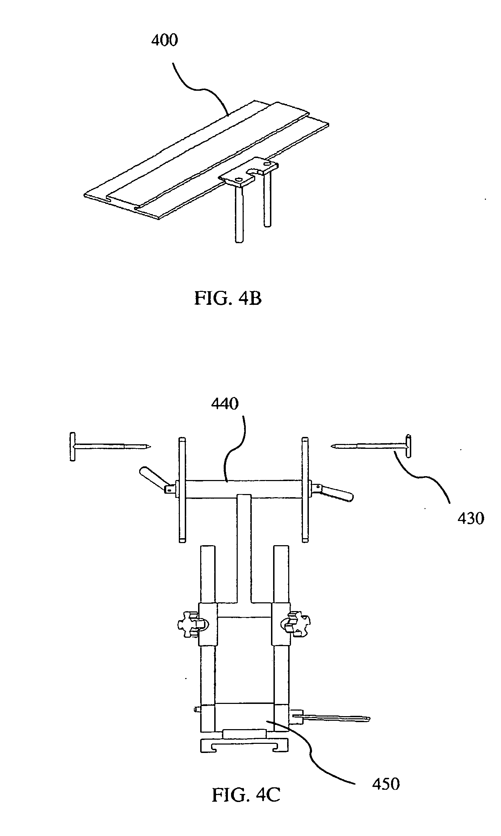

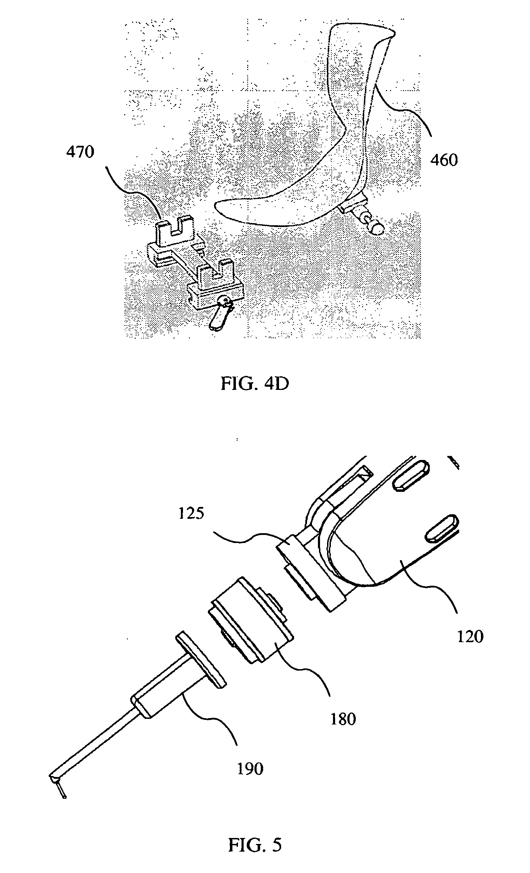

In a preferred embodiment of the invention, the system comprises a limb fixation device (see **FIGS. 4A, 4B, 4C** and **4D)** to ensure the immobility of the leg during the procedure. This limb fixation device allows an immobilization of leg at two levels: at the level of the ankle with a toothed rack **(FIG. 4D);** at the level of the knee with two pins screwed on femoral or tibial epiphysis **(FIG. 4C).**

**FIG. 4B** shows the main plate **400** of the limb fixation device. Main plate **400** is fixed on the operating table with two clamps **300.** The knee fixation part **410** and the ankle fixation part **420** can slide along the main plate **400** and be locked in place by screws.

**FIG. 4C** is a front view of the means of immobilizing patient's leg at the level of the knee. Knee rests on the support bar **440.** As bones are exposed in a knee replacement surgery, two pins **430** are screwed either in the femoral epiphysis or in the tibial epiphysis. The position of the support bar **440** can be adjusted vertically and locked with two knobs. The orientation can be adjusted from 0 to 90° by rotating around the main axis **450** and locked with one knob. The whole system can slide along the plate.

**FIG. 4D** illustrates the means of immobilizing patient's leg at the level of the ankle. Patient foot and ankle are rigidly fixed with surgical tape or other sterile means to lock the foot in the boot **460.** The boot **460** is adapted to be clamped in a carriage **470** that can slide along the main plate **400** and be locked in place with a knob.

Both parts of the limb fixation device (ankle part and knee part) are independent but are used in combination to guarantee immobilization of the lower limb during the procedure.

In a preferred embodiment of the invention, control unit **130** can set the robot arm **120** in a cooperative mode in which a user is able to move the robot arm **120** manually by grabbing it by its final part. With reference to **FIG. 5,** the system of the present invention comprises a force sensor **180** mounted to the robot arm mounting flange **125.** Force sensor **180** is adapted to receive a tool like the pointing tool **190.** When the user grabs the tool and tries to move it in a direction, the control unit **130** receives efforts measured by the force sensor **180** and combines them with the position of the robot arm **120** to generate the movement desired by the user.

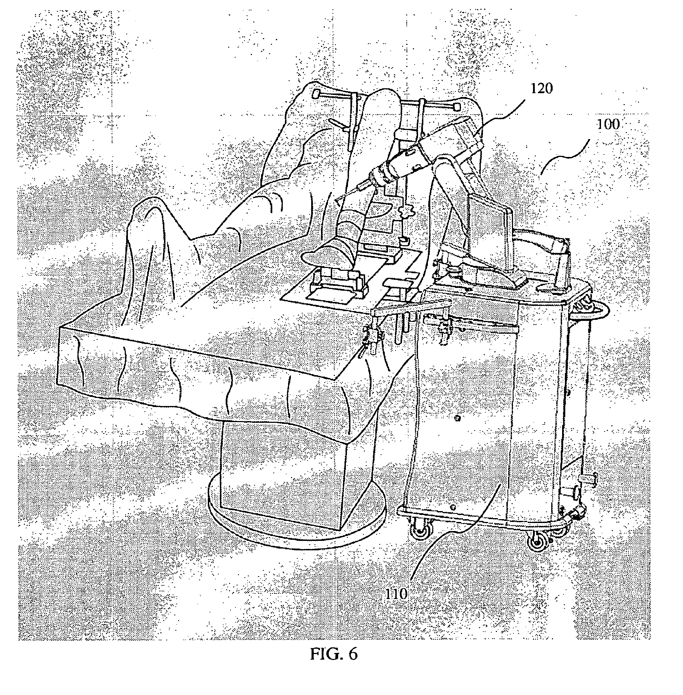

Once the robotized device has been fixed to the operating table, the first step of the procedure is collecting anatomical landmarks on the patient. These anatomical landmarks are known by the surgeon. For example, in a TKR procedure, the malleoluses, the internal part of tibial tuberosity, the middle of the spines and the tibial plateaus are collected on the tibia; the notch middle point, the distal and posterior condyles and the anterior cortex are collected on the femur. **FIG. 6** illustrates positions of the patient and of the robotized device **100** at the beginning of the landmarks collection step for a TKR procedure.

During the landmarks collection step, the control unit **130** sets the robot arm **120** in cooperative mode and indicates through the display monitor **140** the anatomical landmarks to acquire. The surgeon moves the pointing tool **190** until being in contact with the required anatomical landmark and validates the acquisition of the point coordinates using the user interface **150.** The control unit **130** then memorizes the coordinates of the point and its anatomical significance.

After the landmarks collection step, the surgeon inputs planning parameters through the user interface **150.** For example, in a TKR procedure, the surgeon chooses the model and the size of the prosthesis components and defines their positions and orientations relative to the mechanical axes of the femur and the tibia. Typical geometric parameters are varus/valgus angle, posterior slope and thickness of resection for the tibia and varus/valgus angle, flexion/extension angle, external rotation and thickness of resection for the femur.

In another embodiment of the invention, control unit **130** comprises a data-processing interface that enables the system to be connected with another computer-assisted surgical system, like a navigation system. Navigation systems work with preoperative images of the bone (CT scan, X-ray, fluoroscopy, etc) or with intra-operative data. In the latter case, they use a 3D reconstruction algorithm based on the digitalization of the bone. Data provided by the navigation system then replaces, or is combined with the landmarks collection step data. Position of the guiding tool may be generated by the navigation system and transmitted to the robotized device in accordance with a predefined communication protocol.

Once the required position of the guide has been generated, the user mounts the guiding tool to the robot arm. Preferably, a pointing and guiding tool is used, so that the user does not need to change the tool between the landmarks collection step and the cutting or drilling step.

The robotized device **100** accurately aligns the guide relative to patient's anatomy, in accordance with surgeon's planning. If the guiding tool is a cutting guide for a saw blade, the robot arm **120** holds it in the chosen cutting plane. If the guiding tool is a drilling guide, the robot arm **120** holds it along the chosen drilling axis.

In a preferred embodiment of the invention, planar cooperative mode can then be activated by the user to restrict movements of the guide in the plane. Similarly, axial cooperative mode restricts movements of the guide along the axis. The user moves the guiding tool to what he/she estimates is the optimal position, as the control unit **130** restricts movements of the robot arm to a plane or an axis. Once this optimal position reached, the control unit **130** stops the robot arm **120** that holds the guiding tool in place. Surgical task like bone cutting or drilling is carried out by the surgeon using a conventional instrument (oscillating saw or surgical drill) through the guide.

In a TKR procedure, the same guiding tool is used for the tibial cut and the five femoral cuts. In a tibial osteotomy procedure, the same guiding tool is used for both tibial cuts.

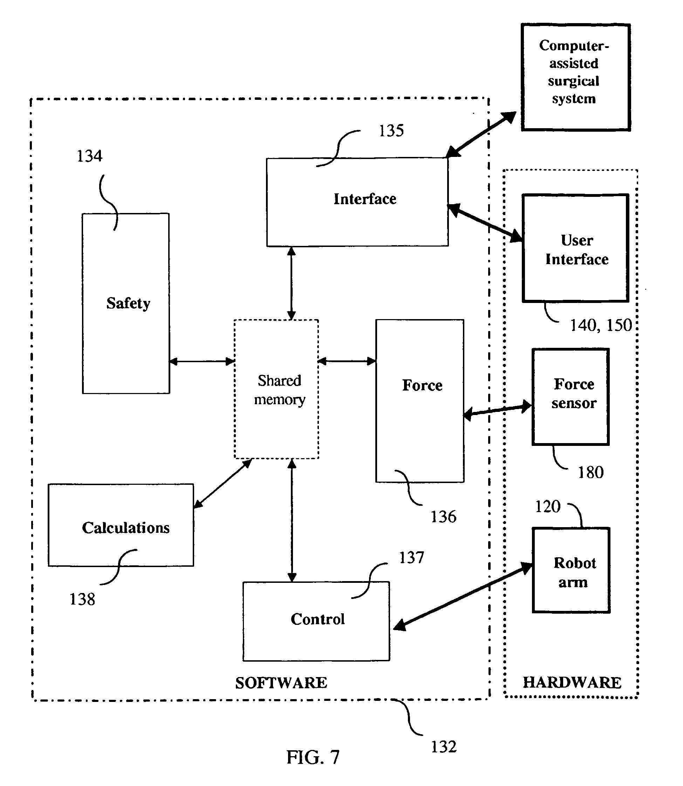

With reference to **FIG. 7,** control unit **130** runs a control software **132,** that exchanges data with elements of the robotized device. Software communicates with the user through the user interface **150** and the display monitor **140.** Software communicates with another computer-assisted surgical system as described above through the data-processing interface. Software communicates with the force sensor **180** to regularly measure the efforts exerted by the user at the tool mounted to the robot arm. Software communicates with the robot arm **120** to control its position.

Control software **132** comprises five independent modules **134** to **138.** Preferably, these modules run simultaneously under a real time environment and use a shared memory to ensure a good management of the various tasks of the control software. Modules have different priorities, safety module **134** having the highest.

Safety module **134** monitors the system status and stops the robot arm **120** when a critical situation is detected (emergency stop, software failure, collision with an obstacle, etc).

Interface module **135** manages the communication between the surgeon and the control software through the user interface **150** and the display screen **140.** Display screen **140** displays a graphical interface that guides the user through the different steps of the procedure. User interface **150** enables the user to have permanent control during the procedure (validating landmarks collection, defining planning parameters, stopping the robot arm if needed, etc).

Force module **136** monitors the forces and torques measured by the force sensor **180.** Force module is able to detect a collision with an obstacle and alert the safety module.

Control module **137** manages the communication with the robot arm **120.** It receives data encoder values of each joint and sends position commands.

Calculations module **138** does all the calculations necessary for the procedure. For example, in a TKR procedure, it reconstructs the mechanical axes of the bones combining anatomical landmarks data and statistical data. It also defines the trajectory of the robot arm **120** using direct and inverse kinematics.

Present invention is not limited by what has been described above. It will be appreciated that various changes can be made therein without departing from the scope of the invention as defined in the appended claims.

## Claims

1. An imageless device for guiding a surgical tool, the device comprising a robot arm and at least one tool wherein the robot arm is adapted to receive at least one of said tool, and a force sensor adapted to be mounted on said robot arm and adapted to receive at least one of said tool,
the device further comprising a means suitable to receive efforts measured by the force sensor, combining said measured efforts with a position of the robot arm and generating the movement of the robot arm desired by the user dependent on the combined effort and position data when operating in a cooperative mode,
a pointing tool received by the robot arm to acquire the coordinates of anatomical landmarks,
a means for manually acquiring and for memorising the co-ordinates of the anatomical landmarks, using the pointing tool,
a means for processing the anatomical landmark co-ordinates thus generating a required position for a guiding tool adapted to guide the surgical tool, and
a means for automatically positioning the guiding tool attached to the robot arm at the required position.

2. The device as claimed in claim 1 wherein the at least one tool comprises a combined pointing and guiding tool.

3. A device according to any of claims 1 or 2, wherein said robot arm presents at least six degrees of freedom.

4. A device according to any of the preceding claims wherein the device further comprises means suited to cause said robot arm to work in a cooperative mode restricting movements of the guide in a plane or along an axis.

5. A device according to any of the preceding claims, that further includes a control monitor and a communication interface adapted to receive surgical planning parameters from a user.

6. A device according to any of the preceeding the claims that further includes a limb fixation device adapted to ensure immobilization of the leg at two levels:
- at the level of ankle with a toothed rack
- at the level of the knee with pins screwed on femoral and tibial epiphysis.

## Patentansprüche

1. Bildlose Einrichtung zum Führen eines chirurgischen Werkzeugs,
wobei die Einrichtung einen Roboterarm und mindestens ein Werkzeug, wobei der Roboterarm geeignet ist, um das mindestens eine Werkzeug aufzunehmen, und einen Kraftsensor umfasst, der geeignet ist, um an dem Roboterarm angebracht zu sein, und geeignet ist, um das mindestens eine Werkzeug aufzunehmen,
wobei die Einrichtung ferner umfasst: ein Mittel, das geeignet ist, um durch den Kraftsensor gemessene Kraftaufwände zu empfangen,
wobei die gemessenen Kraftaufwände mit einer Position des Roboterarms kombiniert werden und bei einem Betrieb in einem kooperativen Modus die von dem Benutzer gewünschte Bewegung des Roboterarms in Abhängigkeit von der Kombination aus Kraftaufwand und Positionsdaten erzeugt wird,
ein Zeigerwerkzeug, das durch den Roboterarm aufgenommen wird, um die Koordinaten anatomischer Merkpunkte zu erhalten,
ein Mittel zum manuellen Erhalten und zum Speichern der Koordinaten der anatomischen Merkpunkte unter Verwendung des Zeigerwerkzeugs,
ein Mittel zum Verarbeiten der Koordinaten der anatomischen Merkpunkte, wodurch eine erforderliche Position für ein Führungswerkzeug erzeugt wird, das geeignet ist, um das chirurgische Werkzeug zu führen, und
ein Mittel zum automatischen Positionieren des an dem Roboterarm befestigten Führungswerkzeugs an der erforderlichen Position.

2. Einrichtung nach Anspruch 1, wobei das mindestens eine Werkzeug ein kombiniertes Zeiger- und Führungswerkzeug umfasst.

3. Einrichtung nach Anspruch 1 oder 2, wobei der Roboterarm mindestens sechs Freiheitsgrade darstellt.

4. Einrichtung nach einem der vorangehenden Ansprüche, wobei die Einrichtung ferner ein Mittel umfasst, das geeignet ist, um zu bewirken, dass der Roboterarm in einem kooperativen Modus arbeitet, der Bewegungen der Führung in einer Ebene oder entlang einer Achse beschränkt.

5. Einrichtung nach einem der vorangehenden Ansprüche, die ferner einen Steuermonitor und eine Kommunikationsschnittstelle umfasst, die geeignet ist, um Parameter einer chirurgischen Planung von einem Benutzer zu empfangen.

6. Einrichtung nach einem der vorangehenden Ansprüche, die ferner eine Gliedmaßenfixierungseinrichtung umfasst, die geeignet ist, um die Immobilisierung des Beins an zwei Ebenen sicherzustellen:
- an der Ebene des Knöchels mit einer Zahnstange
- an der Ebene des Knies mit in die Femur- und Tibiaepiphyse geschraubten Stiften.

## Revendications

1. Dispositif sans image pour guider un outil chirurgical, le dispositif comprenant un bras de robot et au moins un outil, ledit bras de robot étant adapté à recevoir ledit au moins un outil, et un capteur de force adapté à être monté sur ledit bras de robot et adapté à recevoir ledit au moins un outil,
le dispositif comprenant en outre des moyens capables de recevoir des efforts mesurés par le capteur de force, de combiner lesdits efforts mesurés avec une position du bras de robot, et de générer le mouvement du bras de robot désiré par l'utilisateur en dépendance de l'effort combiné et des données de position lorsqu'il fonctionne dans un mode de coopération,
un outil de pointage reçu par le bras de robot pour acquérir des coordonnées de repères anatomiques,
des moyens pour acquérir manuellement et pour mémoriser les coordonnées des repères anatomiques en utilisant l'outil de pointage,
des moyens pour traiter les coordonnées des repères anatomiques en générant ainsi une position requise pour un outil de guidage adapté à guider l'outil chirurgical, et
des moyens pour positionner automatiquement l'outil de guidage attaché au bras de robot à la position requise.

2. Dispositif selon la revendication 1, dans lequel ledit au moins un outil comprend un outil combiné de pointage et de guidage.

3. Dispositif selon l'une quelconque des revendications 1 et 2, dans lequel ledit bras de robot présente au moins six degrés de liberté.

4. Dispositif selon l'une quelconque des revendications précédentes, dans lequel le dispositif comprend en outre des moyens appropriés pour amener ledit bras de robot à travailler dans un mode de coopération en restreignant les mouvements du guide dans un plan ou le long d'un axe.

5. Dispositif selon l'une quelconque des revendications précédentes, incluant en outre un écran de contrôle et une interface de communication adaptée à recevoir des paramètres de planification chirurgicale depuis un utilisateur.

6. Dispositif selon l'une quelconque des revendications précédentes, incluant en outre un dispositif de fixation de membre adapté à assurer l'immobilisation de la jambe à deux niveaux :
-- au niveau de la cheville avec une crémaillère, et
-- au niveau du genou avec des broches vissées sur l'épiphyse fémorale et l'épiphyse tibiale.
